# EUROPEAN PATENT APPLICATION

(11) **EP 3 427 579 A1**
(43) Date of publication of application: **16.01.2019**
(21) Application number: 17763548.9
(22) Date of filing: 07.03.2017
(51) Int. Cl.: A01K 29/00, A01K 13/00, A01K 15/00, G06Q 50/22

(54) **COMPANION ANIMAL MANAGEMENT APPARATUS AND METHOD**

(30) Priority: 09.03.2016 CN 201610133381; 03.01.2017 KR 20170000571
(71) Applicant: Walkbrain Co., Ltd., Seongdong-gu Seoul 04793 (KR)
(72) Inventor: CHOI, Chunggwang, Gwangju 61117 (KR)
(74) Representative: Peterreins Schley
(86) International application number: PCT/KR2017/002464
(87) International publication number: WO 2017/155287

(57) **Abstract**

Disclosed is a companion animal management apparatus. A companion animal management apparatus according to an embodiment of the present invention may comprise: a monitoring and control unit for the real-time monitoring of the behavior of a companion animal; a standard data storage unit for storing standard data generated on the basis of the normal behaviors of the companion animal; an owner treatment data storage unit for storing owner treatment data generated on the basis of the behaviors performed by an owner with respect to the abnormal behaviors of the companion animal; a data comparison unit for retrieving real-time monitoring data on the behaviors of the companion animal obtained by the monitoring and control unit and the standard data within the standard data storage unit, and for determining whether the monitoring data is consistent with the standard data to determine whether the behavior of the companion animal is normal or abnormal; a data matching unit for retrieving the real-time monitoring data on the behavior of the companion animal obtained by the monitoring and control unit and the owner treatment data within the owner treatment data storage unit and for obtaining the owner treatment data for the real-time behavior of the companion animal from the monitoring data and the owner treatment data, if the data comparison unit determines that the behavior of the companion animal is abnormal; and an application unit for retrieving and applying the owner treatment data for the real-time behavior of the companion animal obtained by the data matching unit. The companion animal management apparatus according to the present invention allows the current behavior of the companion animal to be obtained in real-time and allows the current behavior to be compared with the standard data. As a result of the comparison, if it is determined that the behavior of the companion animal is abnormal, the owner can retrieve data on the corresponding behavior and proceed with an interaction with the companion animal. Further, the present invention can realize the effect as if the owner is beside the companion animal, thereby preventing the occurrence of any physical or psychological problems in the companion animal.

## Description

### Technical Field

The present invention relates to a companion animal management apparatus and method and, more particularly, to a technology for observing and analyzing a behavior of a companion animal, and for managing the companion animal.

### Background Art

In recent years, many people live single, and generally, a feeling of loneliness is exacerbated due to their longer single life span.

In an effort to solve this problem, some people overcome the feeling of loneliness by raising companion animal.

However, most people who raise companion animals have jobs, and thus the time spent everyday with their companion animals is very short.

Therefore, the companion animal has to spend a lot of time alone, and thus many companion animals have problems such as autism or depression.

Furthermore, the problems are stressful to the companion animals and owners of the companion animals, particularly in South Korea, when a companion animal not covered by medical insurance uses an animal clinic, the economic loss of companion animal owner increases.

In the related art, some techniques for solving the problems are proposed in Korean Patent Application Publication No. 10-2014-0146806, and Korean Patent Application Publication No. 10-2008-0094117. However, the related art techniques are configured only to input a voice of the owner of a companion animal or a schedule of the companion animal, or to transmit preset simple information, so it is difficult to understand the behavior of the companion animal in real-time, and thus the companion animal management was limited.

### [Documents of Related Art]

### [Patent Documents]

(Patent Document 1) Korean Patent Application Publication No. 10-2014-0146806
(Patent Document 2) Korean Patent Application Publication No. 10-2008-0094117

### Disclosure

### Technical Problem

Accordingly, the present invention has been made keeping in mind the above problems occurring in the related art, and an object of the present invention is to provide a companion animal management apparatus and method capable of detecting abnormal behavior of a companion animal by understanding the behavior of the companion animal in real-time, and of preventing physical and psychological problems that may arise with the companion animal by quick handling of the detected abnormal behavior of the companion animal.

### Technical Solution

The companion animal management apparatus according to the present invention includes:
a monitoring and control unit for monitoring a behavior of a companion animal in real-time;
a standard data storage unit for storing standard data generated on the basis of a normal behavior of the companion animal;
an owner treatment data storage unit for storing owner treatment data generated on the basis of a behavior performed by an owner with respect to an abnormal behavior of the companion animal;
a data comparison unit for retrieving real-time monitoring data for the behavior of the companion animal obtained by the monitoring and control unit and the standard data stored in the standard data storage unit, and for determining whether the behavior of the companion animal is normal or abnormal by comparing the real-time monitoring data to the standard data;
a data matching unit for retrieving the real-time monitoring data for the behavior of the companion animal obtained by the monitoring and control unit and the owner treatment data stored in the owner treatment data storage unit, and for obtaining owner treatment data for the real-time behavior of the companion animal by using the real-time monitoring data and the owner treatment data, when the data comparison unit determines that the behavior of the companion animal is abnormal; and
an application unit for retrieving and applying the owner treatment data for the real-time behavior of the companion animal obtained by the data matching unit.

Preferably, the apparatus further includes a standard data extraction unit, wherein the standard data extraction unit analyzes past behaviors of the companion animal in a constant time period set by the passage of time through a progress of a time comparative analysis method, and extracts analyzed data, so that the standard data extraction unit generates the standard data that is compared to the real-time monitoring data for the behavior of the companion animal obtained by the monitoring and control unit.

Preferably, the apparatus further includes a client unit, wherein the client unit is interconnected with each of the data comparison unit, the data matching unit, and the application unit, and receives and performs an instruction that the owner of the companion animal wants for the abnormal behavior of the companion animal.

Preferably, the monitoring and control unit includes at least one or more of: an audio monitoring and control module for monitoring a sound of the companion animal and obtaining audio data of the companion animal;
a video monitoring and control module for monitoring an image of the companion animal and obtaining video data of the companion animal; and
an environment parameter monitoring and control module for obtaining parameters depending on an environment of a place where the companion animal is located.

Preferably, the environment parameter monitoring and control module includes at least one or more of: a temperature monitoring and control module for monitoring a temperature in the environment of the place where the companion animal is located;
a humidity monitoring and control module for monitoring humidity in the environment of the place where the companion animal is located;
an illumination monitoring and control module for monitoring illumination in the environment of the place where the companion animal is located;
an oxygen content monitoring and control module for monitoring an oxygen content in the environment of the place where the companion animal is located;
an air quality monitoring and control module for monitoring air quality in the environment of the place where the companion animal is located;
a water level monitoring and control module for monitoring a water level in the environment of the place where the companion animal is located;
a microorganism content monitoring and control module for monitoring a microorganism content in the environment of the place where the companion animal is located;
a protein content monitoring and control module for monitoring a protein content in the environment of the place where the companion animal is located;
a salinity monitoring and control module for monitoring salinity in the environment of the place where the companion animal is located; and
an acid alkali concentration monitoring and control module for monitoring acid-alkali concentration in the environment of the place where the companion animal is located.

Preferably, the video monitoring and control module includes an obstruction structure member, a recording structure member, a control structure member, and a signal detection structure member, wherein the signal detection structure member and the obstruction structure member are connected with the control structure member, and the obstruction structure member physically covers the recording structure member.

Preferably, the apparatus further includes a companion animal behavior typifier unit, wherein the companion animal behavior typifier unit has therein a sensor module, and the sensor module is interconnected with the data matching unit.

Preferably, the sensor module includes at least one or more of a pressure sensor, a vibration sensor, and a sound sensor.

A companion animal management method according to the one aspect of the present invention includes:
monitoring, by a monitoring and control unit, a behavior of a companion animal to obtain monitoring data of the companion animal; comparing, by a data comparison unit, the monitoring data to standard data, and determining that the behavior of the companion animal is normal when the monitoring data agrees with the standard data, and determining that the behavior of the companion animal is abnormal when the monitoring data does not agree with the standard data; obtaining, by a data matching unit, owner treatment data matched to the monitoring data when the data comparison unit determines that the behavior of the companion animal is abnormal; and retrieving and applying the owner treatment data obtained by the data matching unit.

### Advantageous Effects

As described above, the present invention may obtain and determine the current behavior of a companion animal in real-time.

Furthermore, the current behavior of the companion animal can be compared with standard data, and when the comparison result is determined to be an abnormal behavior, the owner of the companion animal can realize the same effect as a feeling that the owner of the companion animal is beside of the companion animal by handling it quickly using the present invention.

Finally, it is possible to prevent physical and psychological problems that may occur with the companion animal.

### Description of Drawings

In the description, accompanying drawings describe preferred embodiments of the present invention, and provide a further understanding of a technical idea of the present invention with the following detailed description of the present invention. Thus, the present invention should not be construed as being limited to only subjects described in the drawings.
FIG. 1 is a block diagram of a companion animal management apparatus in accordance with an embodiment of the present invention.
FIG. 2 is a flowchart of a companion animal management method in accordance with an embodiment of the present invention.

### Best Mode

Hereinbelow, to aid to understand the present invention, motional advantages of the present invention, and an object achieved by a practice of the present invention, reference should be made to the accompanying drawings that are illustrative of preferred embodiment of the present invention and the description in the drawings.

Specific structural and functional descriptions of embodiments of the present invention disclosed herein are only for illustrative purpose of the embodiments according to the concept of the present invention, so the embodiments according to the concept of the present invention may be embodied in many different forms.

Further, the present invention should not be construed as being limited to the embodiment described hereinbelow, but should be construed as covering modifications, equivalents, alternatives that may be included within the spirit and technical scope of the present invention.

The terminology used herein is for the purpose of describing particular embodiments only and is not intended to limit the present invention.

Hereinbelow, the preferred embodiment of the present invention will be described in detail with reference to the accompanying drawings. The same reference numerals in the drawings will refer to the same or like elements or parts.

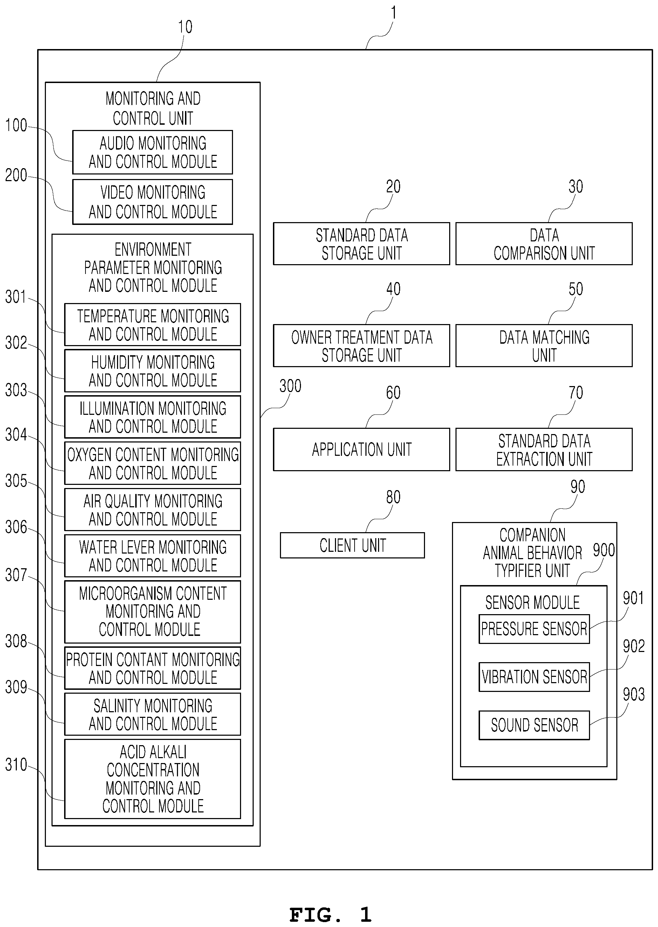

FIG. 1 is a block diagram illustrating the configuration of a companion animal management apparatus according to an embodiment of the present invention, and the apparatus will be described with reference to FIG. 1.

As shown in FIG. 1, the companion animal management apparatus according to the embodiment of the present invention includes a monitoring and control unit 10, a standard data storage unit 20, a data comparison unit 30, an owner treatment data storage unit 40, a data matching unit 50, an application unit 60, a standard data extraction unit 70, a client unit 80, and a companion animal behavior typifier unit 90.

The monitoring and control unit 10 may monitor the current behavior of the companion animal in real-time using a general camera, etc.

The standard data storage unit 20 may store standard data of the behavior of the companion animal. Here, the standard data may be changed as time passes as will be described hereinbelow.

The standard data storage unit 20 may store data of a normal behavior of the companion animal, and may be configured in a general database form.

The data of the normal behavior of the companion animal may agree with the standard data when the owner of the companion animal or a companion animal expert observes the behavior of the companion animal, and determines that the behavior of the companion animal is normal. The standard data may be stored in the standard data storage unit 20.

Past behaviors of the companion animal in a constant time period set by the passage of time may be analyzed by adopting a time comparative analysis method, and the data may be extracted to be set to the standard data that is compared with the current behavior of the companion animal.

For example, when the behavior of the companion animal agrees with the normal behavior of the companion animal when amount of activity within a timed period reaches a value, the value is used as standard data of amount of activity and stored in the standard data storage unit 20.

Thus, the constant standard data is presented for all other behavior of the companion animal.

The standard data may be obtained through routine observation and experience.

Also, various standard data may be set in accordance with various companion animals, various time periods, for example, a season, an age of the companion animal, etc.

It is not necessary to maintain and protect data artificially by automatically updating the standard data.

Through interconnecting the data comparison unit 30 with each of the monitoring and control unit 10 and the standard data storage unit 20, the data comparison unit 30 retrieves real-time monitoring data for the current behavior of the companion animal obtained by the monitoring and control unit 10, and the standard data in the standard data storage unit 20. At the same time, the data comparison unit 30 may determine whether the current behavior of the companion animal agrees with standard data by comparing the real-time monitoring data for the current behavior of the companion animal to the standard data, through the determination, and may determine whether the behavior of the companion animal is normal or abnormal.

The owner treatment data storage unit 40 may store the owner treatment data, and may be configured in a general database form.

The owner treatment data is generated on the basis of a behavior performed by an owner with respect to an abnormal behavior of the companion animal.

For example, when the owner of the companion animal comforts the companion animal exhibiting anxious behavior by voice, the owner treatment data may soon become audio data that the owner of the companion animal uses to comfort the companion animal.

Also, the abnormal behavior of the companion animal may include that hearing an excessive number of loud noises produced by companion animal during the specified time period, continuous excess of the number that do not confirm on presence of the companion animal during the specified time period, the behavior of the companion animal that is obviously slow or fast, the behavior of biting an object that has large volume or moving the object by the companion animal, and an environment parameter (variable) out of a preset environment parameter (variable).

Various owner treatment data may correspond to the various abnormal behaviors of the companion animal, at the same time, stored in the owner treatment data storage unit 40.

Through connecting the data matching unit 50 with each of the monitoring and control unit 10 and the owner treatment data storage unit 40, the data matching unit 50 retrieves real-time monitoring data for the present behavior of the companion animal obtained by the monitoring and control unit 10, and the owner treatment data in the owner treatment data storage unit 40. At the same time, the data matching unit 50 obtains the owner treatment data for the current behavior of the companion animal by a one-to-one correspondence relationship established between the monitoring and control unit 10 and the owner treatment data storage unit 40.

The data matching unit 50 may retrieve a determination result of the data comparison unit 30 by connecting with the data comparison unit 30, and when the current behavior is abnormal behavior, find the owner treatment data corresponding to the abnormal behavior.

The application unit 60 may retrieve the data found by connecting the data matching unit 50, and may apply the owner treatment data.

For example, when the owner treatment data is in the form of audio, the application unit 60 may be implemented as an audio player unit, and broadcast the audio.

Also, when the owner treatment data is in the form of video, the application unit 60 may be implemented as a video player unit, and may broadcast the video.

In order words, in the present invention, the application performed by the application unit 60 may be interpreted as meaning to include an audio or video broadcast as a way of performing the interaction with the companion animal by using the data found by the data matching unit 50.

As mentioned above, the abnormal behavior of the companion animal is processed in real-time to maintain the interaction between the companion animal and the owner of the companion animal through monitoring the current behavior of the companion animal. Also, the owner can realize how to control the abnormal behavior of the companion animal to prevent problems that may occur when the owner is not beside the companion animal and cannot monitor the behavior of the companion animal in real-time, and further the time period during which the owner cannot treat the animal immediately continues for a long time.

The standard data extraction unit 70 may be applied with each data of the monitoring and control unit 10 and the standard data storage unit 20.

Also, for the real-time monitoring data for the current behavior of the companion animal, the past behavior of the companion animal in a constant time period set by the passage of time may be analyzed through the progress of the time comparative analysis in the standard data extraction unit 70, and the past behavior data of the companion animal may be extracted, and may be used as the standard data compared to the current behavior of the companion animal.

At the same time, the standard data may be sent to the standard data storage unit 20 may be recognized as the standard data and be stored in the standard data storage unit 20.

For example, when the set time is 3 days, the behavior analysis is performed on the basis of the behavior of previous 3 days to analyze the behavior of the 4th day of the companion animal.

For example, when analyzing whether the behavior of the 4th day of the companion animal is normal or abnormal, the behaviors of the 1st, 2nd, and 3rd days as the standard data are compared with the behavior of the 4th day.

When analyzing whether the behavior of the 5th day of the companion animal is normal or abnormal, the behaviors of the 2nd, 3rd, and 4th days as the standard data are compared with the behavior of the 5th day.

In this way, the automatic update according to the extraction of the standard data and the passage of time is realized.

Since the behavior of the companion animal is influenced by a variable according to the passage of time such as a changing of a season and aging of the companion animal, the normal behavior of the companion animal, in other words, the standard data, may change with passage of time such as the changing of a season and the aging of the companion animal.

For example, in summer, the behavior of the companion animal is relatively slow because weather is relatively hot, and in spring, the behavior of the companion animal is relatively active because weather is adequate for the companion animal.

Therefore, the standard data adapted to spring is not used in summer.

The standard data may change with seasonal changes.

Another example, when the age of the companion animal is relatively young, the behavior of the companion animal is relatively active, when the age of the companion animal becomes old, the behavior of the companion animal slows down.

Therefore, the standard data used when the age is relatively young is not adapted to the companion animal when the age is relatively old.

The standard data may be changed in accordance with changing in the age of companion animal.

Also, depending on various companion animals, the standard of normal behavior of the companion animal may be diversified.

For example, some companion animals like to move, and some companion animals like to stay quiet.

In other word, the standard data for the same behavior of various companion animals may not be same.

For this reason, by using the companion animal management apparatus of the present invention, the standard data may reflect normal behavior that the companion animal exhibits with the passage of time with the standard data obtained by the time comparison analysis for the current behavior of the companion animal, and changes in the behavior according to the changing of season, the aging of the companion animal, and the changes of the companion animal.

Further, when the standard data is compared with the current behavior of the companion animal, the comparison result may be made more accurately.

According to the present invention, since the monitoring data in constant time period is used as the standard data, the time period is continuously updated with the passage of time, and the standard data may be continuously updated with the passage of time.

Therefore, automatic updating of the standard data may be realized because the problem of artificially updating the standard data can be solved.

The data comparison unit 30 or the data matching unit 50 may be applied with the data of the client unit 80.

The results determined by the data comparison unit 30 and the results obtained by the data matching unit 50 are sent to the client unit 80. Thus, the owner of the companion animal can confirm whether the behavior of the companion animal is normal or abnormal, and confirm the owner treatment data.

The client unit 80 may be applied with the data of the application unit 60.

The client unit 80 may allow the owner of the companion animal to send directly the owner treatment data for the abnormal behavior of the companion animal to the application unit 60 due to the connection with the application unit 60.

The client unit 80 may input and receive the behavior and the data that the owner of the companion animal wants, and may process the behavior and the data through the connection with the application unit 60. Also, the client unit 80 may perform an instruction that the owner of the companion animal wants for the abnormal behavior of the companion animal by receiving the instruction.

Therefore, the owner of the companion animal may realize more accurate control for the abnormal behavior.

When the application unit 60 cannot receive the instruction data sent from the client unit 80, the owner treatment data stored in the owner treatment data storage unit 40 is retrieved.

For example, the owner treatment data is presented in the form of audio or video by the application unit 60 to comfort the companion animal.

Specifically, the audio or the video data that the owner records and stores by using an audio player or a video player is broadcasted.

The monitoring and control unit 10 may include at least one or more of an audio monitoring and control module 100, a video monitoring and control module 200, and an environment parameter monitoring and control module 300.

The audio monitoring and control module 100 may monitor a sound of the companion animal by an audio device, etc., and obtain audio data of the companion animal.

The video monitoring and control module 200 may monitor a video of the companion animal by a camera, etc., and obtain video data of the companion animal.

The environment parameter monitoring and control module 300 may obtain a parameter (variable) depending on a place environment where the companion animal is located.

The environment parameter monitoring and control module 300 may include: a temperature monitoring and control module 301, a humidity monitoring and control module 302, an illumination monitoring and control module 303, an oxygen content monitoring and control module 304, an air quality monitoring and control module 305, a water level monitoring and control module 306, a microorganism content monitoring and control module 307, a protein content monitoring and control module 308, a salinity monitoring and control module 309, and an acid alkali concentration monitoring and control module 310.

The temperature monitoring and control module 301 may monitor a temperature in the environment of the place where the companion animal is located.

The humidity monitoring and control module 302 may monitor humidity in the environment of the place where the companion animal is located.

The illumination monitoring and control module 303 may monitor illumination in the environment of the place where the companion animal is located, by using a lighting device, and may activate the lighting device automatically, when the illumination in the environment of the place where the companion animal is located is equal to or less than a predefined value.

The oxygen content monitoring and control module 304 may monitor oxygen content in the environment of the place where the companion animal is located.

The air quality monitoring and control module 305 may monitor air quality in the environment of the place where the companion animal is located.

The water level monitoring and control module 306 may monitor a water level in the environment of the place where the companion animal is located.

The microorganism content monitoring and control module 307 may monitor the microorganism content in the environment of the place where the companion animal is located.

The protein content monitoring and control module 308 may monitor the protein content in the environment of the place where the companion animal is located.

The salinity monitoring and control module 309 may monitor salinity in the environment of the place where the companion animal is located.

The acid alkali concentration monitoring and control module 310 may monitor the acid alkali concentration in the environment of the place where the companion animal is located.

Especially, the illumination monitoring and control module 303 may grasp illumination in the environment of the place where the companion animal is located by recognizing brightness of a camera image, and activate the lighting device, so the companion animal may be kept comfortably in a room without darkness.

The environment parameter (variable) such as temperature, humidity, oxygen content, air quality, water level, microorganism content, illumination, protein content, salinity, acid alkali concentration may affect the health of the companion animal.

Therefore, when the monitoring for the parameter (variable) described above is performed and the monitored parameter (variable) is not equal to the predefined value, the control for the parameter (variable) may be performed by the application unit 60.

Therefore, the companion animal may be kept continuously in a comfortable environment, and a problem such as illness or death of the companion animal may be prevented.

Also, the environment parameter (variable) may be sent to a mobile phone of the owner to allow that the owner can know conditions of the place where the companion animal is located.

Also, since the problem of a strange environment may be solved, the companion animal may be kept in the comfortable environment for a long time.

When the owner knows that the companion animal is located in a strange environment, the owner can control the environment parameter monitoring and control module 300 through the client unit 80, and adjust the environment parameter (variable).

When a circumstance such as a blackout occurs, the control for the monitoring and control unit 10 may not be possible.

In this case, through the client unit 80, the owner can be connected to a service system, and can activate the monitoring and control unit by home service of service personnel.

The video monitoring and control module 200 may include an obstruction structure member, a recording structure member, a control structure member, and a signal detection structure member. The signal detection structure member and the obstruction structure member may be connected with the control structure member, and the obstruction structure member may physically cover the recording structure member.

Wherein, the video monitoring and control module 200 may monitor the behavior of the owner when the owner comes home, in addition to the monitoring and control unit 10 processing the monitoring of the behavior of the companion animal.

However, many people do not want that their behaviors monitored and exposed to the outside.

Therefore, to solve the problem described above, the embodiments of the present invention wherein the signal of the owner is detected by the signal detection structure member, and when a signal of the owner is detected, for example, the owner came home already, and the owner is within a monitoring scope of the monitoring and control unit 10, the signal detection structure member sends a signal to the control structure member. The control structure member operates the obstruction structure member after receiving the signal, and allows the obstruction structure member to cover the recording structure member, thus causing the recording structure member to lose a recording function.

Therefore, the video monitoring and control is not performed. Further, since the owner is at home, the owner can monitor the behavior of the companion animal directly, and since the owner can interact with the companion animal, the owner does not have to use the companion animal management apparatus of the present invention.

However, when the owner is at home but out of the monitoring scope of the monitoring and control unit 10, the owner needs to use the companion animal management apparatus of the present invention. Thus, the obstruction structure member is not operated, and the recording structure member may perform the recording function, so the video monitoring and control and the management function may be realized.

The owner can block by the set cutoff function, and open all functions by using a mobile phone when the owner is at home and needs to use the monitoring and control unit 10.

The companion animal management apparatus of the present invention may further include a companion animal behavior typifier unit 90. The companion animal behavior typifier unit 90 may have a sensor module. The sensor module may be applied with the data of the data matching unit 900. Accordingly, the companion animal may proactively interact with the owner.

When the companion animal uses the companion animal behavior typifier unit 90, the signal of typifier used by the companion animal may be sent to the data matching unit 50 through the sensor module. Upon receiving the signal, the data matching unit 50 may retrieve the owner treatment data, and return the owner treatment data corresponding to the behavior when the companion animal uses the typifier.

Next the application unit 60 may realize the interaction between the owner and the companion animal by using the owner treatment data.

Especially, the owner treatment data corresponding to the behavior when the companion animal uses the typifier may be stored in advance, and at the same time, the training for the companion animal may be performed. Further, the owner can provide the treatment corresponding to the behavior when the companion animal uses the typifier. Therefore, it enables clarifying the results when the companion animal uses the typifier.

The companion animal can proactively use the typifier after obtaining the treatment of the owner, and thus the companion animal can proactively perform the connection and the interaction with the owner.

The companion animal can send directly the signal using the typifier to the client unit 80 of the owner when the companion animal interacts with the owner. Thus, the owner can connect and interact directly with the companion animal.

Here, the companion animal behavior typifier of the companion animal behavior typifier unit 90 may be an object with an arbitrary shape such as a spherical shape. The companion animal can use the typifier through the behavior of pushing the typifier or biting the typifier by the mouth of the companion animal.

Moreover, while using the management apparatus, when the sensor module responds, the signal may be sent by a Bluetooth, ZigBee, WIFI, or infrared. Also, the signal may be sent to the client unit 80 of the owner through the companion animal management apparatus of the present invention (for example, a mobile phone, a personal digital assistant (PDA), a computer, etc.).

The sensor module 900 may include a pressure sensor 901, a vibration sensor 902, and a sound sensor 903.

The sensor module 900 may include various types of sensors, when they are able to send the signal to the data matching unit 50 or the client unit 80.

For example, when the companion animal wants to be connected with the owner, the companion animal sends a signal through the pressure sensor 901 (by pressing), the vibration sensor 902 (by bumping), and a sound sensor 903 (by comparing with preset audio), and thus an administrator may determine the intention of the companion animal through the signal.

Functions performed by the monitoring and control unit 10, the standard data storage unit 20, the data comparison unit 30, the owner treatment data storage unit 40, the data matching unit 50, the application unit 60, the standard data extraction unit 70, the client unit 80, and the companion animal behavior typifier unit 90 may be realized by a central processing units (CPU) or an application program interface (API), and may be interlocked with a smart terminal.

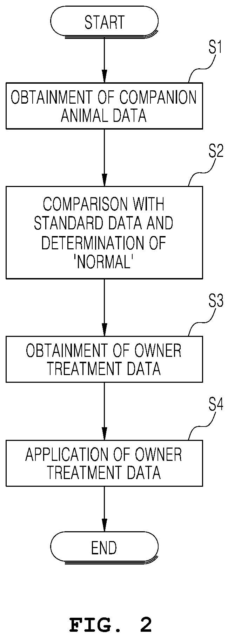

FIG. 2 is flowchart illustrating the companion animal management method according to an embodiment of the present invention.

As shown in FIG. 2, the companion animal management performed by the companion animal management apparatus 1 according to the embodiment of the present invention may include following steps of:
monitoring a current behavior of the companion animal to obtain the current behavior data of the companion animal (S1),
comparing the current behavior data to the standard data, and determining that the behavior of the companion animal is normal when the current behavior data agrees with the standard data, and entering a next procedure (S2),
when the current behavior data does not agree with the standard data, determining that the behavior of the companion animal is abnormal, and performing step S3,
obtaining the owner treatment data matched to the current behavior data (S3), and
applying the owner treatment data (S4).

Here, the current behavior of the companion animal may be a behavior to go around a circle, and the data for the current behavior may be a number of turns, for example, 10 times.

Storing the standard data for the behavior of the companion animal is the storing the normal behavior data of the companion animal.

When the owner or a companion animal expert observes the behavior of the companion animal and determines that the behavior is normal, the normal behavior data of the companion animal is used as standard data and may be stored in the standard data storage unit 20.

For example, when the behavior of the companion animal agrees with the normal behavior of the companion animal when the amount of activity within a predetermined time period reaches a value, the value is used as standard data of the amount of activity and stored in the standard data storage unit 20.

Constant standard data may be provided for all other behaviors of the companion animal. The standard data may be provided through regular observation and experience.

Also, various standard data may be set in accordance with various companion animals and various time periods, for example, season, an age of the companion animal, etc. The standard data is automatically updated continuously.

The owner treatment data is provided for the treatment performed by the owner for an abnormal behavior of the companion animal.

For example, when the owner of the companion animal wants to comfort the companion animal exhibiting anxious behavior by voice, the owner treatment data becomes audio data that the owner of the companion animal can use to comfort the companion animal. Therefore, the owner treatment data and the abnormal behavior of the companion animal may correspond one-to-one.

When the behavior of the companion animal is abnormal, the corresponding owner treatment data may be retrieved and processed to realize the control for the companion animal.

The standard data is past behavior data within a specified time period. Also, the specified time period is rolling updated in real-time.

Since the behavior of the companion animal is influenced by a variable according to the passage of time such as changing of a season and aging of the companion animal, the normal behavior of the companion animal, in other words, the standard data is influenced by the variable related to the passage of time such as the changing of the season and the aging of the companion animal.

Therefore, the standard data may change with time passes. Moreover, the comparison result may be clarified when the standard data is compared with the current behavior of the companion animal.

The standard data may be artificially updated continuously.

In the embodiment, the real-time monitoring data of the companion animal is analyzed by the standard data extraction unit 70, and may be stored in the standard data storage unit 20.

Since the monitoring data in constant time period is used as the standard data, the time period is continuously updated with the passage of time, also the standard data may be continuously updated with the passage of time.

Therefore, it is possible to solve the troublesome problem of artificially updating of standard data. Also, the standard data may be assured to update automatically continuously with time passage.

S3 is a step of establishing relationship between the abnormal behavior data of the companion animal and the owner treatment data, and may include a process of retrieving the owner treatment data corresponding to the current behavior data.

Wherein the owner treatment data may be stored, and may be sent directly to the data matching unit 50 through the client unit 80 of the owner, and may be performed through the application unit 60.

When the owner sends the owner treatment data through the client unit 80, the process of establishing the relationship between the abnormal behavior data of the companion animal and the owner treatment data is specifically that when the abnormal behavior data of the companion animal is sent to the client unit 80, the owner sends the owner treatment data to an application part through the client unit 80 on the basis of the abnormal behavior data of the companion animal.

When the application unit 60 cannot receive the instruction data sent by the client unit 80, the owner treatment data stored in the owner treatment data storage unit 40 is retrieved.

For example, the owner treatment data is presented in the form of audio or video data for the owner by the application unit 60 to comfort the companion animal.

Specifically, the audio or the video data that the owner records and stores by using an audio player or a video player is broadcasted.

Also, the abnormal behavior of the companion animal may include continuous hearing of an excessive number of loud noises given by the companion animal during a specified time period, continuous excess of the number of times that the existence of the companion animal is not confirmed in the specified time period, the behavior of the companion animal that is obviously slow or fast, excess of time of staying of the companion animal at a food container over a preset time, the behavior of biting an article that has large volume or moving the article by the companion animal, and an environment parameter (variable) out of a preset environment parameter (variable)

Here, the loud noise given by the companion animal may be barking of a very large dog when the dog hears a sound of door knocking or a stranger comes into the home.

In the expression the companion animal staying at a food container over a preset time, the length of the time means that the companion animal stays at the food container too long or too short. In other word, when the companion animal stays longer than the preset time, the companion animal is hungry, and when the companion animal stays shorter than the preset time, the companion animal has a loss of appetite.

The loss of appetite or hunger of the companion animal is all the abnormal behavior of the companion animal.

The owner treatment data may include audio data, video data, and/or a method of controlling the environment parameter (variable) about the abnormal behavior of the companion animal.

Here, the audio data may be audio recorded by the owner or music for the companion animal downloaded by the owner. Also, the video data may be video recorded by the owner or a video for the companion animal downloaded by the owner.

The audio recorded by the owner, the music for the companion animal downloaded by the owner, the video recorded by the owner, and the video for the companion animal downloaded by the owner may be recorded or downloaded in advance, and may be stored and used directly as required.

The application in step S4 may include broadcasting audio by using an audio player, broadcasting video by using a video player, and/or adjusting the setting of environment parameter (variable) by the owner using an environment parameter controller.

Here, the audio data may be audio recorded by the owner or music for the companion animal downloaded by the owner. Also, the video data may be video recorded by the owner or video for the companion animal downloaded by the owner.

The audio recorded by the owner or the music for the companion animal downloaded by the owner and the video recorded by the owner or the video for the companion animal downloaded by the owner may be recorded or downloaded in advance, and may be stored and used directly as required.

According to the kind of abnormal behavior of the companion animal, only one data may be used or several data may be used, and only one environment parameter (variable) may be controlled or several environment parameters (variable) may be controlled.

Before performing step S1, the following process may be added; detecting whether the owner returned home, and when the owner returned home, detecting whether the owner is in the monitoring scope of the monitoring and control unit 10, and when the owner is in the monitoring scope, turning off the apparatus, and otherwise, performing step S1.

When monitoring about the companion animal, the video monitoring and control module may monitor the behavior of the owner when the owner comes home, beside that the monitoring and control unit 10 performs the monitoring about the behavior of the companion animal.

However, many people do not want that their behaviors are monitored and exposed to the outside. Thus, to solve the problem described above, the procedure of detecting whether the owner returns home is performed first in the embodiment of the present invention, so it may be assured that the behavior of the owner is not monitored.

Furthermore, when the owner returns the home and is in the monitoring scope of the monitoring and control unit 10, the monitoring and control unit 10 may monitor for the behavior of the owner. Therefore, the monitoring and control management may be prevented by turning off the monitoring and control unit 10.

Also, when the owner has already returned home, but is not in the monitoring scope of the monitoring and control unit 10, the companion animal management apparatus and method of the present invention may be used.

In this situation, the method provided by the embodiment of the present invention may be performed.

Moreover, when the owner is at home, the owner may monitor the behavior of the companion animal directly, and interact with the companion animal directly.

Here, to detect whether the owner has returned the home may be performed by the following process:
checking whether the monitoring and control unit 10 and the client unit 80 carried by the owner are connected to WIFI in the house at the same time or are identical with the location of preset GPS in the house. When they are identical, it may be determined that the owner has already returned home, and otherwise, it may be determined that the owner has not returned home yet.

All or part of the process related to the embodiment described above may be completed by instructing to a related hardware through a program

The program is stored in a searchable storage media in a computer, and all or part of the various embodiments described above may be performed. The computer may be, for example, a personal computer, a server, a network device, a smart mobile terminal, a smart home unit, a wearable smart device, and a vehicle-mounted smart device. The storage media may be, for example, a random-access memory (RAM), a read-only memory (ROM), a disk, a tape, a video tape, a flash memory, a universal serial bus memory, a external hard disk, a memory card, a memory stick, a network server memory, and a network cloud memory

Likewise, steps such as S1, S2, S3, and S4 described above may be performed by CPU and API, and may be interlocked with a smart terminal.

The present invention is described in detail with reference to preferred embodiment. The present invention, however, is not limited to only the example embodiments set forth herein, and those skilled in the art will appreciate that the present invention can understand that the technical scope of the present invention affects within available ranges to transform or modify without departing from the gist of the present invention the patent right of the present invention.

## Claims

1. A companion animal management apparatus, comprising:
a monitoring and control unit for monitoring a behavior of a companion animal in real-time;
a standard data storage unit for storing standard data generated on the basis of a normal behavior of the companion animal;
an owner treatment data storage unit for storing owner treatment data generated on the basis of a behavior performed by an owner with respect to an abnormal behavior of the companion animal;
a data comparison unit for retrieving real-time monitoring data for the behavior of the companion animal obtained by the monitoring and control unit and the standard data stored in the standard data storage unit, and for determining whether the behavior of the companion animal is normal or abnormal by comparing the real-time monitoring data to the standard data;
a data matching unit for retrieving the real-time monitoring data for the behavior of the companion animal obtained by the monitoring and control unit and the owner treatment data stored in the owner treatment data storage unit, and for obtaining owner treatment data for real-time behavior of the companion animal by using the real-time monitoring data and the owner treatment data, when the data comparison unit determines that the behavior of the companion animal is abnormal; and
an application unit for retrieving and applying the owner treatment data for the real-time behavior of the companion animal obtained by the data matching unit.

2. The apparatus of claim 1, further comprising a standard data extraction unit, wherein the standard data extraction unit analyzes past behaviors of the companion animal in constant time period set by time passage through a progress of a time comparative analysis method, and extracts the analyzed data, so that the standard data extraction unit generates the standard data that is compared to the real-time monitoring data for the behavior of the companion animal obtained by the monitoring and control unit.

3. The companion animal management apparatus of claim 1, further comprising a client unit,
wherein the client unit is interconnected with each of the data comparison unit, the data matching unit, and the application unit, and receives and performs an instruction that the owner of the companion animal wants for the abnormal behavior of the companion animal.

4. The companion animal management apparatus of claim 1, wherein the monitoring and control unit includes at least one or more of:
an audio monitoring and control module for monitoring a sound of the companion animal and obtaining audio data of the companion animal;
a video monitoring and control module for monitoring an image of the companion animal and obtaining video data of the companion animal; and
an environment parameter monitoring and control module for obtaining parameters depending on an environment of a place where the companion animal is located.

5. The companion animal management apparatus of claim 4, wherein the environment parameter monitoring and control module includes at least one or more of:
a temperature monitoring and control module for monitoring a temperature in the environment of the place where the companion animal is located;
a humidity monitoring and control module for monitoring humidity in the environment of the place where the companion animal is located;
an illumination monitoring and control module for monitoring illumination in the environment of the place where the companion animal is located, and for activating a lighting device automatically when the illumination in the environment of the place where the companion animal is located is equal to or less than a predefined value;
an oxygen content monitoring and control module for monitoring an oxygen content in the environment of the place where the companion animal is located;
an air quality monitoring and control module for monitoring air quality in the environment of the place where the companion animal is located;
a water level monitoring and control module for monitoring a water level in the environment of the place where the companion animal is located;
a microorganism content monitoring and control module for monitoring a microorganism content in the environment of the place where the companion animal is located;
a protein content monitoring and control module for monitoring a protein content in the environment of the place where the companion animal is located;
a salinity monitoring and control module for monitoring salinity in the environment of the place where the companion animal is located; and
an acid alkali concentration monitoring and control module for monitoring an acid alkali concentration in the environment of the place where the companion animal is located.

6. The companion animal management apparatus of claim 4, wherein the video monitoring and control module includes an obstruction structure member, a recording structure member, a control structure member, and a signal detection structure member, wherein the signal detection structure member and the obstruction structure member are connected with the control structure member, and the obstruction structure member physically covers the recording structure member.

7. The companion animal management apparatus of claim 1, further comprising a companion animal behavior typifier unit,
wherein the companion animal behavior typifier unit has therein a sensor module, and the sensor module is interconnected with the data matching unit.

8. The companion animal management apparatus of claim 7,
wherein the sensor module includes at least one or more of a pressure sensor, a vibration sensor, and a sound sensor.

9. A companion animal management method, comprising:
monitoring, by a monitoring and control unit, a behavior of a companion animal to obtain monitoring data of the companion animal;
comparing, by a data comparison unit, the monitoring data to standard data, and determining that the behavior of the companion animal is normal when the monitoring data agrees with the standard data, and determining that the behavior of the companion animal is abnormal when the monitoring data does not agree with the standard data;
obtaining, by a data matching unit, owner treatment data matched to the monitoring data when the data comparison unit determines that the behavior of the companion animal is abnormal; and
retrieving and applying the owner treatment data obtained by the data matching unit.
